# EUROPÄISCHE PATENTANMELDUNG

(11) **EP 0 705 581 A1**
(43) Veröffentlichungstag der Anmeldung: **10.04.1996**
(21) Anmeldenummer: 95115778.3
(22) Anmeldetag: 06.10.1995
(51) Int. Cl.: A61F 5/00, A61F 5/01

(54) **Sprunggelenkorthese**

(30) Priorität: 07.10.1994 DE 4435878
(71) Anmelder: Wilhelm Josef Hefele GmbH, D-87600 Kaufbeuren (DE)
(72) Erfinder: Wilhelm Josef Hefele GmbH, D-87600 Kaufbeuren (DE)
(74) Vertreter: Hübner, Hans-Joachim, Dipl.-Ing.

(57) **Zusammenfassung**

Eine Sprunggelenk-Orthese (10) weist zwei Seitenwände (12,14) auf, die in der oberen Hälfte der Orthese durch eine Vorderwand (16) zu einem relativ steifen Formkörper verbunden sind. An den Hinterkanten (20) der Seitenwände (12, 14) ist die Orthese (10) elastisch aufweitbar, um sie an den Fuß eines Patienten anlegen zu können. Mittels Spanngurten lassen sich die Seitenwände (12,14) eng an das Bein des Patienten anlegen. Die unten frei endenden Seitenwände (12, 14) bilden eine Bodenöffnung (26), deren Spaltbreite mittels eines Spannbandes (31) einstellbar ist, um die Seitenwände (12, 14) unter elastischer Verformung eng an den Patientenfuß anlegen zu können.

## Beschreibung

Die Erfindung betrifft eine Sprunggelenkorthese mit den Merkmalen des Oberbegriffes von Patentanspruch 1.

Eine derartige Sprunggelenkorthese ist aus der EP-A-0269946 bekannt. Die Bodenplatte besteht aus einer starren Bodenwand, die einstückig mit den Seitenwänden verbunden ist. Die Orthese bildet damit einem in einem Längsschnitt formstabilen U-förmigen Bügel. Die Wände bestehen aus einem faserverstärkten Kunststoff, insbesondere einem Polyacryl-Harz mit eingelegter Kohlefasermatte. Innenseitig ist die Orthese mit einem Perlongewebe ausgekleidet. Diese Sprunggelenkorthese hat sich wegen der hohen Formstabilität und Verwindungssteifigkeit in der Praxis bewährt.

Die US-A-4,844,094 zeigt eine aus Einzelteilen bestehende Orthesen-Anordnung mit zwei separaten Seitenwänden, einer Bodenplatte sowie Gurten und Spannband zum Anlegen der Einzelteile am Bein des Probanden. Auch wenn die Gurte und das Bodenspannband sehr fest gezogen werden, können sich die Seitenwände relativ zueinander und zur Bodenplatte verschieben. Zwar läßt sich eine gewisse Stützwirkung des Sprunggelenkes wie bei einer steifen Bandage erzielen, jedoch ist eine unerwünschte Bewegung des Sprunggelenkes nicht ausgeschlossen.

Der eingangs genannte Stand der Technik vermeidet diesen Nachteil dank des Schaftes, bei dem die Seitenwände mit der Vorderwand einstückig verbunden sind. Diese Vorderwand dient zur Anlage des Schienenbeins. Die während der Gangphase auftretenden Kräfte werden vom causalen Schienenteil aufgefangen und über die kollateralen Anteile auf den distalen Unterschenkelbereich übertragen. Das Sprunggelenk ist dadurch fixiert. Allerdings erfordert die Sprunggelenkorthese nach der EP-A-0269 946 eine anatomisch exakte Anpassung. Es müssen mehrere Größen auf Lager gehalten werden, von denen eine passende Größe ausgewählt, durch Erwärmung verformbar gemacht und an den Patientenfuß angepaßt wird. Während der Tragezeit der Orthese verändert sich aber die Form des Fußes, sodaß eine Nachstellung wünschenswert wäre. Diese ist aber bei der Orthese nach der EP-A-026 9946 nicht möglich.

Aufgabe der Erfindung ist es daher, eine Sprunggelenkorthese der eingangs genannten Art unter Beibehaltung deren Vorteile nachstellbar auszubilden und für mehrere Fußgrößen geeignet zu machen.

Diese Aufgabe wird bei einer Sprunggelenkorthese mit den Merkmalen des Patentanspruches 1 gelöst.

Während die Sprunggelenkorthese nach der EP-A-0269 946 ein nahezu starrer Formkörper ist, der sowohl im vertikalen Längsschnitt als auch - auf Höhe der Vorderwand - im horizontalen Querschnitt U-förmig ausgebildet ist, enden bei der erfindungsgemäßen Orthese die Seitenwände mit parallelen Bodenkanten. Da das bodenseitig angeformte Verbindungsjoch fehlt, lassen sich die unten frei endenden Seitenwände durch elastische Verbiegung formen. Diese relative Beweglichkeit ist nur mit einem Freiheitsgrad vorhanden, d.h. die beiden Seitenwände können im unteren Bereich aufeinander zubewegt werden, um den Abstand der Seitenwände im Fußbereich zu verringern. Eine relative Verschiebung der Seitenwände in Höhenrichtung oder in horizontaler Vor-bzw. Rückwärtsrichtung ist dagegen dank der verbleibenden Vorderwand praktisch ausgeschlossen. Die Beweglichkeit der Seitenwände in ihrem unteren Bereich in horizontaler Querrichtung ermöglicht es nun aber in einfacher Weise, die Orthese an die jeweilige Fußform des Patienten exakt anzupassen. Dies kann täglich neu geschehen. Außerdem eignet sich eine Orthesengröße für verschiedenen Fußgrößen, da die Fuß-und Knöchelbreite des Schaftes verstellbar ist, sodaß die Lagerhaltung reduziert wird.

Eine ausgewählte Schaftgröße kann somit mit verschieden weiten Bodenplatten kombiniert werden, die als Einfachteile mit feingestufter Breite auf Lager gehalten oder auch individuell ausgeschnitten werden können. Die Bodenplatte muß sich nicht notwendigerweise nach hinten bis unter die Ferse des Patienten erstrecken, sondern kann zu einem schmalen Steg schrumpfen, der sich vor der Ferse des Patienten befindet. In einer vereinfachten Ausführungsform kann die Bodenplatte gänzlich entfallen, sodaß der Fuß des Patienten auf dem Spannband ruht. Die Nachstellbarkeitsfunktion des Schaftes geht dadurch nicht verloren, jedoch wird an Tragekomfort eingebüßt. Jedenfalls fällt auch eine Sprunggelenkorthese ohne Bodenplatte ausdrücklich unter die Erfindung.

Vorzugsweise bildet jedoch die Bodenplatte definierte Einstellpositionen für die unteren Enden der Seitenwände, indem sich diese an der Bodenplatte anlegen und durch das Spannband in diesen Festpositionen fixiert werden. Mit dieser Ausgestaltung erhält die Orthese nahezu dieselbe Starrheit wie die einstückige bekannte Orthese, allerdings mit dem Vorteil, daß durch Auswechseln der Bodenplatten gegen solche geringfügig anderer Breite Feinjustagen möglich sind.

Eine weitere Ausgestaltung der Erfindung ist darin zu sehen, daß die Bodenplatte einen flachen Querkanal aufweist, der vom Spannband durchsetzt wird, das entweder um die Bodenkanten der Seitenwände herum zu deren Außenflächen geführt ist, oder mit dem Querkanal fluchtende Schlitze in den Seitenwänden durchsetzt. Der Querkanal kann bodenseitig offen oder auch geschlossen sein.

Die Verstellbarkeit der erfindungsgemäßen Orthese im Fußbereich ermöglicht auch eine individuelle Anpassung der Orthese vom Patienten selbst, wobei mit zunehmender Genesung des Patienten, die Orthese auch zunehmend lockerer eingestellt werden kann. Diese Möglichkeit trägt zur Komforterhöhung maßgeblich bei.

Schließlich besteht noch eine Weiterbildung der Erfindung darin, daß die Seitenwände und die Vorderwand innerseitig mit einer einstückigen, diese Wände wenigsten angenähert vollflächig abdeckenden, einen geschlossenen Hohlraum bildenden luftdichten Hülle belegt sind, die einen einzigen zentralen Aufblasanschluß hat, der durch eine Öffnung in der Vorderwand nach außen geführt ist und ein von außen betätigbares Abschlußorgan aufweist. Der Aufblasanschluß sitzt - auf die horizontale Außenkontur der Orthese bezogen - mittig, d.h. ist mit der höchsten Stelle der vorderen Ristöffnung vertikal ausgerichtet. Mit einer Handpumpe in Form eines Gummiballes kann diese Hülle nach Anlegen der Orthese aufgeblasen werden, um die Orthese am Fuß zu fixieren. Solche aufblasbaren Auskleidungen sind zwar an sich bekannt, jedoch bedarf es z.B. bei der Orthese nach der DE-A-29 13 606 zweier getrennter Hüllen für jede Seitenwand und es müssen demgemäß auch zwei Aufblasstutzen vorgesehen werden. Da das Aufblasen beider Hüllen nacheinander erfolgen muß, tritt in der Praxis der Nachteil auf, daß der Aufblaszustand beider Hüllen unterschiedlich ist, was sich auf den einwandfreien Sitz der Orthese nachteilig auswirkt. Der erfindungsgemäße zentrale Aufblasstutzen für die einstückige Hülle vermeidet diesen Nachteil und stellt sicher, daß die Luftpolster an beiden Seitenwänden eine identische Ausdehnung erfahren.

Anhand der Zeichnung, die ein Ausführungsbeispiel zeigt, wird die Erfindung näher beschrieben.

Es zeigt:

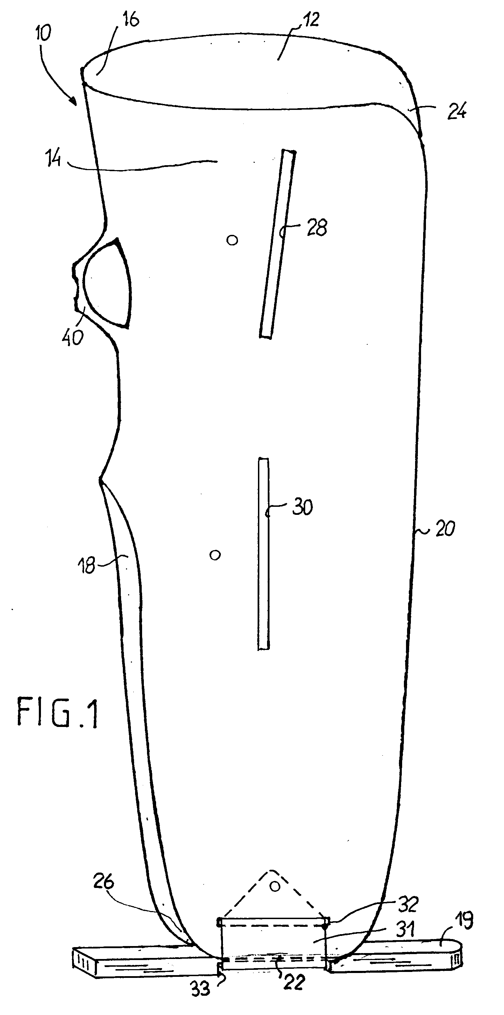

- Fig. 1: eine von lateral her gesehene Ansicht einer ersten Ausführungsform einer Sprunggelenkorthese,
- Fig.2: eine Ansicht von medial einer Sprunggelenkorthese, die sich von der Orthese nach Figur 1 durch Einbau eines Luftkissens unterscheidet, und

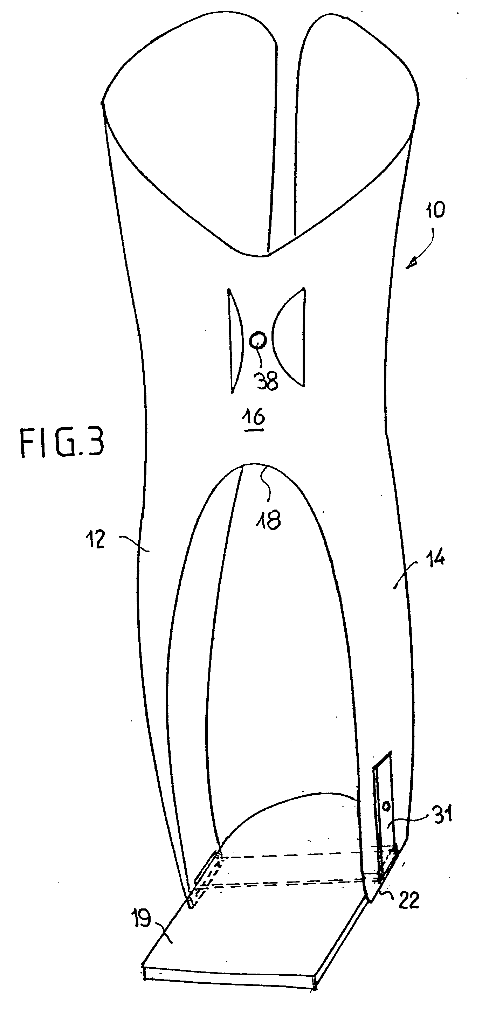

- Fig.3: eine perspektivische Vorderansicht der Orthese nach Fig.1
Eine Sprunggelenkorthese 10 hat zwei längliche Seitenwände 12, 14 gleicher Länge und eine Vorderwand 16, die die Seitenwände 12, 14 im oberen Bereich der Orthese verbindet. Unterhalb der Vorderwand 16 befindet sich eine Ristöffnung 18, die in Vorderansicht etwa halbellyptisch ausgebildet ist. Die vertikalen Längskonturen der Seitenwände bilden wenigstens angenähert Geraden. Im Horizontalquerschnitt sind die Seitenwände 12, 14 gewölbt. Auch die Vorderwand 16 ist gewölbt, jedoch mit kleineren Krümmungsradien als die Seitenwände. Die Übergänge der Vorderwand 16 zu den Seitenwänden 12, 14 sind stetig. Die Seitenwände 12, 14 weisen im wesentlichen geradlinig verlaufende Hinterkanten 20 auf, die mittels Übergangsbögen in Oberkanten und Bodenkanten 22 der Seitenwände übergehen. Zwischen den Hinterkanten 20 der Seitenwände 12, 14 wird ein Spalt 24 von wenigstens angenähert gleicher Spaltbreite gebildet.

Die Wände der Orthese 10 bestehen aus relativ steifem Material, das gewisse Federeigenschaften hat, sodaß der Spalt 24 elastisch aufweitbar ist, um die Orthese 10 am Fuß anzulegen. Die Seitenwände 12, 14 schnappen dann elastisch in ihre Ausgangslage zurück und umschließen den Unterschenkel, wobei jedoch der ursprüngliche Spalt 24 erhalten bleibt. Die Vorderwand 16 hält die Relativpositionen der Seitenwände 12, 14 aufrecht, wobei eine translatorische Parallel-Verschiebung der Seitenwände 12, 14 zueinander und eine relative Verdrehung ausgeschlossen sind. Außer der schon beschriebenen Aufweitung der Orthese mit Veränderung der Breite des rückwärtigen Spaltes 24 läßt sich auch die Breite der zwischen den Bodenkanten 22 der Seitenwände 12, 14 gebildeten Bodenöffnung 26 verändern. Durch Einstellung dieser beiden Spalt-bzw. Öffnungsbreiten läßt sich die Orthese 10 den Formen des Beines und des Fußes des Patienten individuell anpassen.

In der Bodenöffnung 26 befindet sich eine Bodenplatte 19, die nach vorn und hinten übersteht. In der spannungsfreien Ausgangsstellung ist die Breite der Bodenöffnung größer als diejenige der Bodenplatte 19.

In den Seitenwänden 12, 14 sind jeweils obere Schlitze 28 zum Hindurchführen und Festlegen eines Spanngurtes vorzugsweise in Form eines Klettbandes vorgesehen, wobei in der lateralen Seitenwand 14 ein solcher Schlitz 28 genügt. Weiterhin sind im mittleren Höhenbereich entsprechende Schlitze 30 in den Seitenwänden vorgesehen, die sich zum größten Teil in dem Höhenbereich der Ristöffnung 18 befinden. Die nicht dargestellten Spanngurte haben eine Breite, die der Schlitzlänge entspricht. Mit einem oberen Spanngurt und einem mittleren Spanngurt läßt sich die Orthese 10 um das Bein des Patienten schließen. Das Schienenbein kommt dann zur Anlage an der Vorderwand 16. Ein bodenseitiges Spannband 31 ist z.B. an der lateralen Seitenwand 14 innenseitig befestigt und tritt aus einem horizonten Schlitz 32 aus, der parallel zur Bodenkante 22 verläuft. Das Spannband läuft dann an der Außenseite der Seitenwand 14 nach unten und um die Bodenkante 22 herum, und weiter in einem bodenseitig offenen Querkanal 33 der Bodenplatte 19 zur Bodenkante 22 der medialen Seitenwand 12 und an deren Außenseite nach oben und durchsetzt die beiden dort vorgesehenen parallelen Führungsschlitze 32 und endet an der Außenseite der Seitenwand 12.

Durch Nachziehen des Bandendes verbiegen sich die Seitenwände 12, 14 in ihren unteren Bereichen elastisch und legen sich eng an den Fuß des Patienten an. Der bodenseitige Verstellbereich der Orthese 10 ist ausreichend um ein und dieselbe Orthese für Füße in einem weiten Fußgrößenbereich einsetzen zu können.

Die Breite der Bodenplatte 19 ist dem Fuß des Patienten individuell angepaßt, sodaß die Seitenwände 12, 14 bis zur Anlage an den Seitenwänden der Bodenplatte 19 herausgezogen werden können. Damit ist die Funktionsstellung der Orthese 10 definiert, in der diese ihren optimalen Sitz am Bein des Patienten hat. Beim häufigen Abnehmen und Wiederanlegen der Orthese 10 kommt der so gewonnenen Reproduzierbarkeit der Funktionsstellung der Seitenwände 12, 14 große Bedeutung zu.

Anstatt das Spannband 31 um die Bodenkanten 22 der Seitenwände 12, 14 herumzuführen, können in diesen dicht benachbart der Bodenkanten angeordnete Schlitze vorgesehen sein, die mit dem Querkanal 33 in der Bodenplatte 19 fluchten.

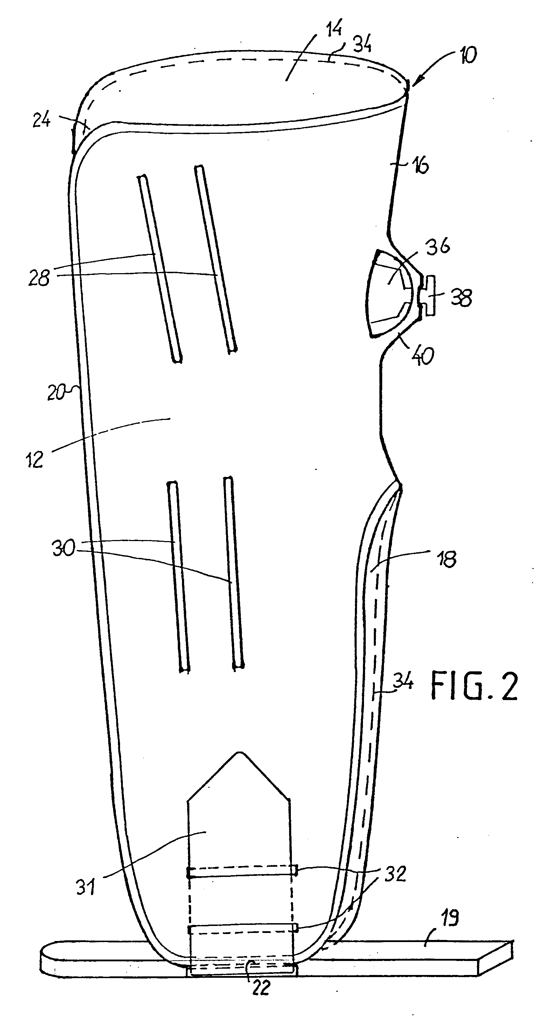

In Fig. 2 ist die gesamte Orthese 10 innenseitig mit einer aufblasbaren Hülle 34 versehen, die sich einstückig längs der Seitenwände 12, 14 und der Vorderwand 16 erstreckt. Die Hülle bildet einen doppelwandigen Zuschnitt aus luftdichtem Material, der randseitig ringsum verschlossen ist, sodaß die Hülle einen geschlossenen Hohlraum bildet. An diesen Hohlraum ist ein Aufblasanschluß 36 angeschlossen, der in einem Abschlußorgan 38 in Form eines Rückschlagventils mündet. Die Vorderwand 16 hat vertikal oberhalb der obersten Stelle der Ristöffnung 18 eine Öffnung, durch die sich der Aufblasanschluß 36 nach außen erstreckt. Ein nach außen gewölbter vorderseitiger Steg 40 deckt die Vorderwandöffnung ab, schützt den Aufblasanschluß 36 und dient zu dessen Lagerung. Mittels einer Handpumpe kann die einstückige Hülle 34 gleichmäßig mit Druckluft beaufschlagt werden, sodaß die Polsterpartien an beiden Seitenwänden 12, 14 gleichzeitig und gleichmäßig aufgeweitet werden.

## Patentansprüche

1. Sprunggelenk-Orthese, mit einem steifen Schaft, der durch zwei Seitenwände (12, 14) und eine diese oberhalb einer vorderseitigen Ristöffnung (18) einstückig verbindende gewölbte Vorderwand (16) gebildet ist, wobei an den Seitenwänden (12, 14) im Höhenbereich der Vorderwand (16) mindestens ein, den rückseitigen Spalt (24) zwischen ihnen überbrückender Spanngurt befestigbar ist und zwischen denen sich bodenseitig eine Bodenplatte (19) als Fußauflage erstreckt, **dadurch gekennzeichnet, daß** der Schaft im Bereich der Fußauflage eine Bodenöffnung (26) mit einer vorgegebenen Ausgangsbreite aufweist, die größer als die Fußbreite des Patienten ist, daß die Bodenplatte (19) ein separates Bauteil bildet und eine Breite hat, die kleiner als die Ausgangsbreite der Bodenöffnung (26) ist, daß die Bodenplatte (19) von einem flexiblen Spannband oder zwei Spannbandabschnitten durchsetzt oder untergriffen wird, dessen oder deren Enden an den Seitenwänden (12, 14) befestigbar sind, und daß die Seitenwände (12, 14) unter elastischer Verformung mittels des Spannbandes (31) in Funktionsstellungen gehalten werden, in denen die Breite der Bodenöffnung (26) kleiner als deren Ausgangsbreite ist.

2. Sprunggelenk-Orthese nach Anspruch 1, **dadurch gekennzeichnet, daß** die Bodenränder (22) der Seitenwände (12, 14) in deren Funktionsstellungen der Bodenplatte (19) dicht benachbart liegen.

3. Sprunggelenk-Orthese nach Anspruch 2, **dadurch gekennzeichnet, daß** die Seitenwände (12, 14) in deren Funktionsstellungen an der Bodenplatte (19) angelegt sind.

4. Sprunggelenk-Orthese nach einem der Ansprüche 1 bis 3, **dadurch gekennzeichnet, daß** die Bodenkanten (22), der Seitenwände (12,14) zu einem wesentlichen Teil in einer gemeinsamen Horizontalebene verlaufen.

5. Sprunggelenk-Orthese nach Anspruch 4, **dadurch gekennzeichnet, daß** die Bodenkanten (22) der Seitenwände (12, 14) mit der Bodenebene der Bodenplatte (19) wenigstens angenähert bündig liegen.

6. Sprunggelenk-Orthese nach einem der Ansprüche 1 bis 5, **dadurch gekennzeichnet, daß** der rückwärtige Spalt (24) des Schaftes wenigstens angenähert eine konstante Breite hat.

7. Sprunggelenk-Orthese nach einem der Ansprüche 1 bis 6, **dadurch gekennzeichnet, daß** die Seitenwände (12, 14) und die Vorderwand (16) innenseitig mit einer einstückigen, diese Wände innenseitig wenigstens angenähert vollflächig abdeckenden, einen geschlossenen Hohlraum bildenden luftdichten Hülle (34) belegt sind, die einen einzigen zentralen Aufblasabschluß (36) hat, der durch eine Öffnung in der Vorderwand (16) nach außen geführt ist und ein von außen betätigbares Abschlußorgan (38) aufweist.

8. Sprunggelenk-Orthese nach Anspruch 7, **dadurch gekennzeichnet, daß** der Aufblasanschluß (36) mit der höchsten Stelle der vorderen Ristöffnung (18) vertikal ausgerichtet ist.
